# EUROPEAN PATENT APPLICATION

(11) **EP 4 278 950 A1**
(43) Date of publication of application: **22.11.2023**
(21) Application number: 23173544.0
(22) Date of filing: 16.05.2023
(51) Int. Cl.: A61B 3/12, A61B 3/14, A61B 3/135, G06T 1/00

(54) **OPHTHALMOLOGIC APPARATUS AND FOCUS DETERMINATION METHOD**

(30) Priority: 17.05.2022 JP 2022080958
(71) Applicant: Topcon Corporation, Tokyo 174-8580 (JP)
(72) Inventor: Fujii, Kohta, Tokyo, 174-8580 (JP); Morishima, Shunichi, Tokyo, 174-8580 (JP)
(74) Representative: Schmitt-Nilson Schraud Waibel Wohlfrom Patentanwälte Partnerschaft mbB

(57) **Abstract**

An ophthalmologic apparatus (1) includes: a spectroscopic member (22) that separates light emitted from a light source (21) into a first spectral component (Ls21) and a second spectral component (Ls22); an optical scanner (51) that guides the first spectral component and the second spectral component to an observation target region (61) of a subject's eye (E) including a plurality of imaging target lines (611) formed by dividing the observation target region in a scanning direction (D1); a line exposure imaging element (33) that includes a plurality of exposure lines (332) capable of detecting return light (Lb) from the observation target region in the light receiving region (331, 331A), each of the exposure lines having an imaging position corresponding to an associated one of the imaging target lines; and a controller (14) that illuminates, when exposure is sequentially performed on two or more of the exposure lines forming a predetermined exposure line group (333) in order, a region of the imaging target lines corresponding to the two or more exposure lines forming the exposure line group with the first spectral component and the second spectral component to perform focus determination based on the result of detection by the exposure lines in the exposure line group.

## Description

The present disclosure relates to an ophthalmologic apparatus and a focus determination method.

There has been conventionally proposed a slit scan fundus camera (an ophthalmologic apparatus) that captures an image of a fundus of a subject's eye. For example, Japanese Patent No. 5736211 describes an ophthalmologic apparatus that moves slit light (illumination light) illuminating the fundus using an optical scanner and captures an image of return light returning from an illuminated region of the fundus illuminated with the moving slit light using a complementary metal-oxide semiconductor (CMOS) imaging element having a rolling shutter function. Accordingly, the ophthalmologic apparatus can acquire a fundus image that is less influenced by scattered light.

Japanese Patent No. 6518054 discloses an ophthalmologic apparatus that illuminates the fundus with a split index light and detects return light of the split index light from the fundus by a detector to perform evaluation and control of the focused state of a fundus camera based on the detection result.

Each of the ophthalmologic apparatuses disclosed in Patent Documents 1 and 2 has an illumination system for evaluation of the focused state and a further illumination system for observation of the subject's eye. This assumably increases the size and cost of the entire apparatus.

In view of the foregoing, it is an object of the present disclosure to provide an ophthalmologic apparatus and a focus determination method that enable observation of a subject's eye and focus evaluation with a simple configuration.

To achieve the object described above, an ophthalmologic apparatus of the present disclosure includes: a spectroscopic member that separates light emitted from a light source into a first spectral component and a second spectral component; an optical scanner that guides the first spectral component and the second spectral component to an observation target region of a subject's eye including a plurality of imaging target lines formed by dividing the observation target region in a scanning direction; a line exposure imaging element that includes a plurality of exposure lines capable of detecting return light from the observation target region in a light receiving region, each of the exposure lines having an imaging position corresponding to an associated one of the imaging target lines; and a controller that illuminates, when exposure operation is sequentially performed on two or more of the exposure lines forming a predetermined exposure line group, a region of the imaging target lines corresponding to the two or more exposure lines forming the exposure line group with the first spectral component and the second spectral component to perform focus determination based on a result of detection by the exposure lines in the exposure line group.

To achieve the object described above, a focus determination method of the present disclosure is a focus determination method for an ophthalmologic apparatus including: a spectroscopic member that separates light emitted from a light source into a first spectral component and a second spectral component; an optical scanner that guides the first spectral component and the second spectral component to an observation target region of a subject's eye including a plurality of imaging target lines formed by dividing the observation target region in a scanning direction; and a line exposure imaging element that includes a plurality of exposure lines capable of detecting return light from the observation target region in a light receiving region, each of the exposure lines having an imaging position corresponding to an associated one of the imaging target lines. The focus determination method includes illuminating, when exposure operation is sequentially performed on two or more of the exposure lines forming a predetermined exposure line group, a region of the imaging target lines corresponding to the two or more exposure lines forming the exposure line group with the first spectral component and the second spectral component to perform focus determination based on a result of detection by the exposure lines in the exposure line group.

The ophthalmologic apparatus and focus determination method described above enable observation of the subject's eye and focus evaluation with a simple configuration.
FIG. 1 is a schematic view illustrating a general configuration of an ophthalmologic apparatus of an embodiment of the present disclosure.
FIG. 2 is a functional block diagram of a control device.
FIG. 3 is an optical path diagram of the ophthalmologic apparatus, schematically showing an optical path of light emitted from a spectroscopic member.
FIG. 4 is an optical path diagram of the ophthalmologic apparatus, schematically showing an optical path of light emitted from a first focus optical system.
FIG. 5 is an operation flowchart of the ophthalmologic apparatus.
FIG. 6 is a diagram showing side views each illustrating a subject's eye and an optical path of illumination light in a focused or unfocused state and front views each illustrating the fundus of the subject's eye illuminated with the illumination light in the focused or unfocused state as viewed from the front (in a P direction).
FIG. 7 is a diagram showing a front view of the fundus, a light receiving region of an imaging element, and an acquired image during focus adjustment.
FIG. 8 is a timing chart of illumination light projected on the fundus and a timing chart of an exposure operation of the imaging element during the focus adjustment.
FIG. 9 is a diagram showing a relationship between an exposure line and the illumination light with the illumination light in the focused or unfocused state.
FIG. 10 is a diagram showing side views each illustrating a subject's eye and an optical path of illumination light during the focus adjustment and front views each illustrating the fundus of the subject's eye illuminated with the illumination light as viewed from the front (in a P direction) during the focus adjustment.
FIG. 11 is a diagram showing a front view of the fundus, a light receiving region of an imaging element, and an acquired image during the focus adjustment.
FIG. 12 is a diagram showing a front view of the fundus, a light receiving region of an imaging element, and an acquired image during slit scan imaging.
FIG. 13 is a timing chart of illumination light projected on the fundus and a timing chart of an exposure operation of the imaging element during slit scan imaging.
FIG. 14 is a diagram showing a relationship between a line profile and a modulation transfer function.

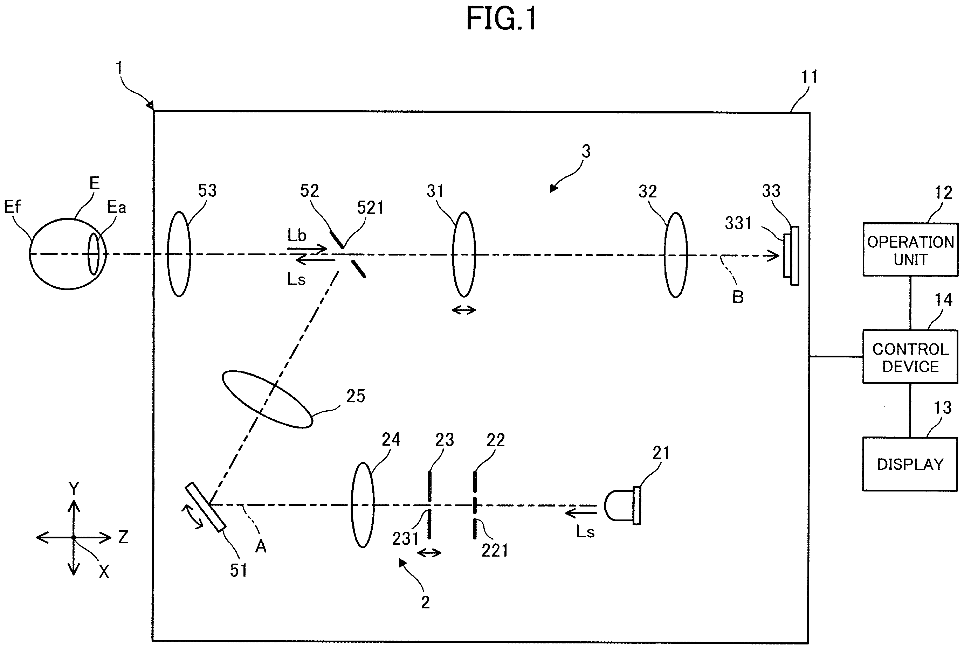

Embodiments of the present disclosure will be described in detail with reference to the drawings. FIG. 1 is a view illustrating an overall configuration of an ophthalmologic apparatus 1. In FIG. 1, an X direction is a left-right direction relative to a subject (a direction of an interpupillary distance between subject's eyes E), a Y direction is an up-down direction, and a Z direction is a front-back direction (also referred to as a working distance direction) which is a near-far direction relative to the subject. In the following description of the ophthalmologic apparatus 1, components and their arrangement are schematically illustrated and may be different from the actual scale for explanatory convenience.

The ophthalmologic apparatus 1 is able to capture an image of the fundus Ef of the subject's eye E by a slit scan method (slit scan imaging). The ophthalmologic apparatus 1 includes an apparatus body 11 that functions as a camera head, an operation unit 12, a display 13, and a control device 14 (controller).

The apparatus body 11 is held by a driving mechanism (not shown) that is manually or automatically movable in the X, Y, or Z direction relative to the subject's eye E. The apparatus body 11 is configured to be movable relative to the subject's eye E for alignment.

The operation unit 12 is capable of receiving inputs for various operations of the ophthalmologic apparatus 1 such as an operation to start the slit scan imaging, an operation of moving the apparatus body 11 relative to the subject's eye E, and an operation of setting the ophthalmologic apparatus 1.

The display 13 may be, for example, a known display such as a liquid crystal display (LCD). The display 13 shows a fundus image, which is an observation image (front image) of the fundus Ef generated by the control device 14, and various setting screens.

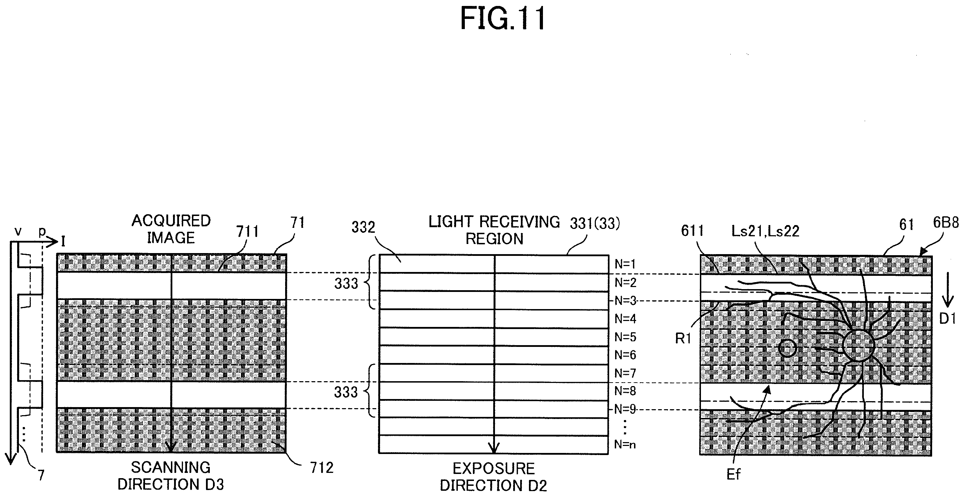

The control device 14 is an arithmetic processing unit such as a computer that executes various kinds of arithmetic processing and control processing. The apparatus body 11, the operation unit 12, and the display 13 are connected to the control device 14 to be able to communicate with the control device 14. For example, the control device 14 integrally controls the operations of the apparatus body 11 and the display 13 based on an operation instruction inputted to the operation unit 12. The control device 14 executes various types of control and processing including: alignment of the apparatus body 11; determination as to whether an illumination system 2 and a light receiving system 3 are focused on the fundus Ef using a line profile 7 (see, e.g., FIG. 11); control of the focus of a first focus optical system 23 and the focus of a second focus optical system 31, slit scan imaging of the fundus Ef by the apparatus body 11; and generation and display of the fundus image.

The configuration of the apparatus body 11 will be described below. The apparatus body 11 includes an illumination system 2 and a light receiving system 3.

The illumination system 2 includes a light source 21, a spectroscopic member 22, a first focus optical system 23, lenses (a first illumination system lens 24, a second illumination system lens 25, and an objective lens 53), an optical scanner 51, and an optical path splitter 52. Note the following: The first focus optical system 23 and a target site (e.g., the fundus Ef) achieve optical conjugation. The control device 14 controls the focus depending on the relative positions of the ophthalmologic apparatus 1 and the subject's eye E and the position of the target site on the subject's eye E.

The light source 21 emits illumination light Ls. The light source 21 includes a light source element that emits, as the illumination light Ls, visible light (e.g., white light) when the slit scan imaging of the fundus Ef is performed and near-infrared light (light in the infrared region) which is less visible to the subject's eye E when adjustment of the focus is performed. The light source 21 may include one or more light source elements. The visible light may also be used for the focus adjustment. Examples of the light source element used for the light source 21 include a laser light emitting element, a light emitting diode (LED), and a fluorescent light emitting element.

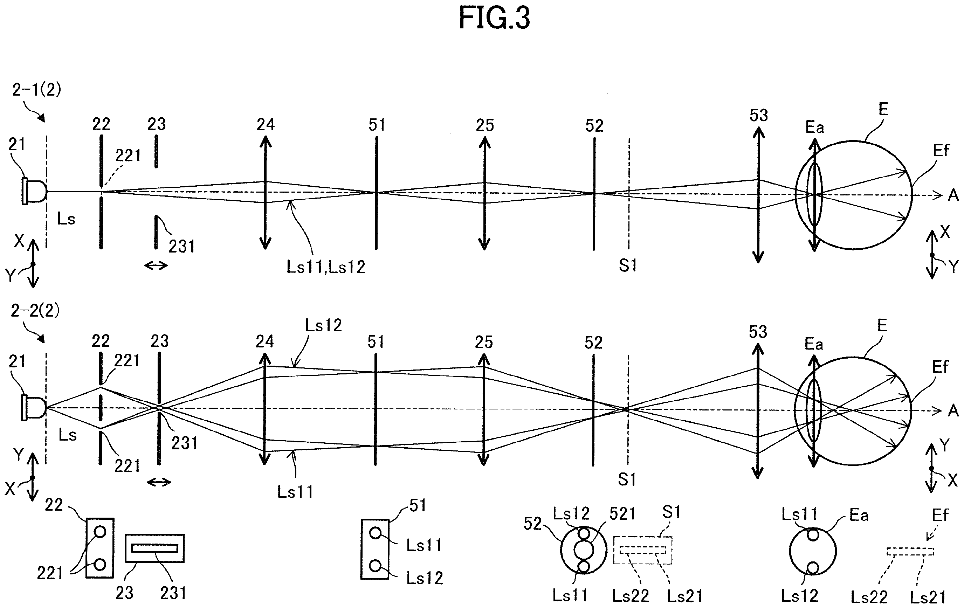

The spectroscopic member 22 has a plurality of separation holes 221 formed into a circular shape and arranged in the Y direction of FIG. 1 (also referred to as a dividing direction) and divides the light emitted from the light source 21. The spectroscopic member 22 is positioned to achieve optical conjugation or quasi-optical conjugation with an anterior segment Ea (the cornea or crystalline lens) of the subject's eye E, the optical scanner 51, and the optical path splitter 52. The separation holes 221 are arranged symmetrically with respect to an optical axis A. In the present embodiment, two separation holes 221 are arranged apart from each other in the Y direction of FIG. 1 (see also FIG. 3 and other figures). The spectroscopic member 22 causes the illumination light Ls emitted from the light source 21 to pass through the two separation holes 221 to separate the illumination light Ls in the Y direction perpendicular to the optical axis A shown in FIG. 1 into a first spectral component (second light component Ls21) and a second spectral component (second light component Ls22).

The first focus optical system 23 has a slit hole 231 that receives the spectral components emitted from the spectroscopic member 22. The slit hole 231 is formed into an elongated rectangular shape (see FIG. 3). The slit hole 231 is disposed on the optical axis A with its longer sides extending in the X direction. The first focus optical system 23 is positioned to achieve optical conjugation or quasi-optical conjugation with the target site of the subject's eye E (the fundus Ef in the present embodiment). Thus, the spectroscopic member 22 and the first focus optical system 23 achieve the optical conjugation at different positions of the eye. The second focus optical system 31 is arranged to be movable along an optical axis B of the illumination light Ls (an axis part of which between the subject's eye E and the optical path splitter 52 is common to the optical axis A) and adjusts the focus of the light receiving system 3 under the control of the control device 14. In the first focus optical system 23, one or more lenses may be movably arranged on the front side and/or rear side of the slit hole 231 on the optical axis A. The adjustment of the focus is not limited to a particular method.

The first illumination system lens 24 collects the illumination light Ls (the first spectral component (Ls21) and the second spectral component (Ls22)) emitted from the slit hole 231 of the first focus optical system 23 and guides the illumination light Ls to the optical scanner 51.

As the optical scanner 51, an optical element such as a galvanometer mirror, a resonant mirror, a polygon mirror, or a microelectromechanical system (MEMS) may be used. The optical scanner 51 has a deflection function, i.e., is able to one-dimensionally deflect (scan) the illumination light Ls entering from the first illumination system lens 24 near the light source 21 so that the illumination light Ls is reflected and guided toward the second illumination system lens 25 downstream of the first illumination system lens 24.

The control device 14 controls the deflection angle or direction of the illumination light Ls deflected by the optical scanner 51. For the slit scan imaging, the optical scanner 51 deflects the illumination light Ls in a direction perpendicular to both the optical axis A of the objective lens 53 (the Z direction in FIG. 1) and the longitudinal direction of the slit hole 231

(the X direction in FIG. 1), i.e., in the Y direction in FIG. 1. Thus, the optical scanner 51 is capable of guiding the illumination light Ls emitted from the first focus optical system 23 so that the illumination light Ls movably illuminates an illuminated region R1 in the target site (e.g., the fundus Ef) of the subject's eye E.

The second illumination system lens 25 collects the illumination light Ls emitted from the optical scanner 51 and guides the illumination light Ls to the optical path splitter 52.

The optical path splitter 52 is a well-known mirror with a hole, i.e., an annular reflector having a substantially circular opening 521 for passing the light. The optical path splitter 52 reflects the illumination light Ls emitted from the second illumination system lens 25 toward the objective lens 53 and allows return light Lb coming from the objective lens 53 to pass through to be guided to the light receiving system 3. The optical path splitter 52 may be a mirror or a splitter of a different shape as long as the optical path splitter 52 is able to split the optical paths of the illumination light Ls and the return light Lb (i.e., is able to guide the illumination light Ls toward the objective lens 53 close to the subject's eye E and guide the return light Lb to the light receiving system 3).

The objective lens 53 allows the illumination light Ls reflected from the optical path splitter 52 to illuminate part of the fundus Ef after passing through the anterior segment Ea (the cornea or crystalline lens) of the subject's eye E. At this time, the illumination light Ls is deflected in the Y direction by the optical scanner 51, allowing the illumination light Ls elongated in the X direction (slit light) to scan the fundus Ef in the Y direction (scanning direction D1). While the illumination light Ls is deflected in the Y direction, the return light Lb from the fundus Ef of the subject's eye E illuminated with the illumination light Ls is guided to the light receiving system 3 through the objective lens 53 and the optical path splitter 52.

The light receiving system 3 includes the objective lens 53, the optical path splitter 52, the second focus optical system 31, a light receiving system lens 32, and an imaging element 33.

The second focus optical system 31 includes one or more lenses (focus lenses) movable along the optical axis B of the return light Lb (the axis part of which between the subject's eye E and the optical path splitter 52 is common to the optical axis A) and adjusts the focus of the light receiving system 3 under the control of the control device 14. Focusing of the light receiving system 3 by the second focus optical system 31 and focusing of the illumination system 2 by the first focus optical system 23 occur in synchronization in accordance with the diopter (visibility) of the subject's eye E. The return light Lb coming from the optical path splitter 52 to the second focus optical system 31 enters the light receiving system lens 32. The second focus optical system 31 may have one or more varifocal lenses instead of the one or more movable focus lenses, and the adjustment of the focus is not limited to a particular method.

The light receiving system lens 32 includes one or more lenses and collects the return light Lb coming from the second focus optical system 31 to the imaging element 33.

For example, a CMOS image sensor is used as the imaging element 33, and the imaging element 33 is disposed to be able to detect the return light Lb from an observation target region of the subject's eye E. The imaging element 33 has a light receiving region 331 that receives the return light Lb from the light receiving system lens 32, and has a rolling shutter function to detect (receive or capture an image of) the return light Lb while changing timing for starting and finishing the exposure performed on predetermined exposure lines 332 one by one in the light receiving region 331. During the slit scan imaging, the imaging element 33 is driven to perform the rolling shutter function by the control device 14 to capture an image of the return light Lb of the illumination light Ls that is deflected by the optical scanner 51 to move in the fundus Ef, and allows the control device 14 to output an imaging signal of the return light Lb.

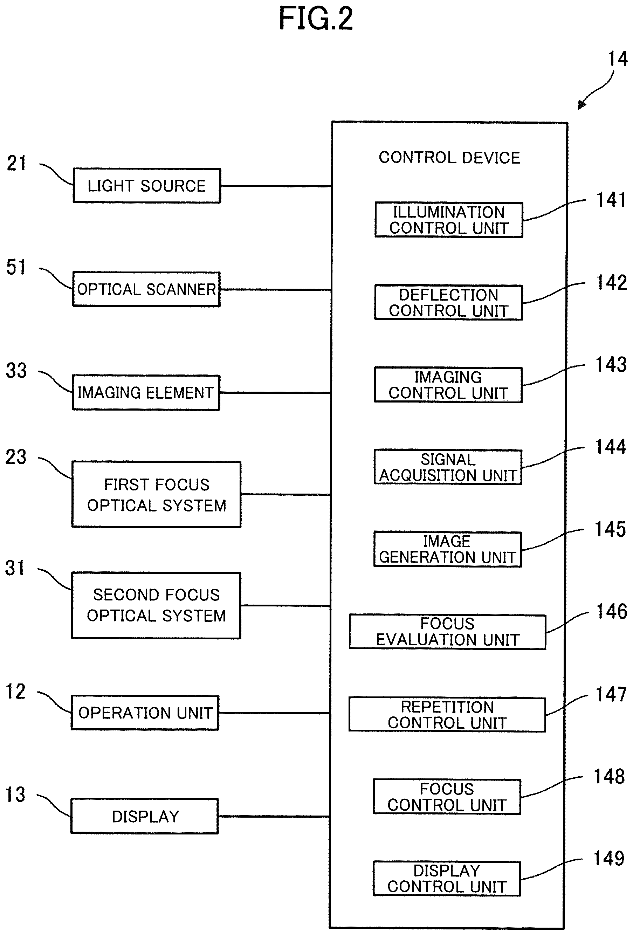

FIG. 2 is a functional block diagram of the control device 14. The functions of the control device 14 are achieved by using various processors. Examples of the various processors include a central processing unit (CPU), a graphics processing unit (GPU), an application specific integrated circuit (ASIC), and programmable logic devices (e.g., simple programmable logic device (SPLD), a complex programmable logic device (CPLD), and a field programmable gate array (FPGA)). The functions of the control device 14 may be achieved by a single processor or a plurality of processors of the same type or different types.

Executing a control program (not shown) allows the control device 14 to function as an illumination control unit 141, a deflection control unit 142, an imaging control unit 143, a signal acquisition unit 144, an image generation unit 145, a focus evaluation unit 146, a repetition control unit 147, a focus control unit 148, and a display control unit 149. Each functional unit of the control device 14 may be implemented by one of software or hardware such as a program, a circuit, a device, or equipment or a combination of the software and the hardware.

The optical path of the illumination system 2 will be described below. FIG. 3 is an optical path diagram of the illumination system 2 showing the optical path of the illumination light Ls (first light components Ls11 and Ls12) passing through the spectroscopic member 22. A plan view 2-1 is shown in the upper part of the drawing, and a side view 2-2 is shown in the middle part. The spectroscopic member 22 allows part of the illumination light Ls emitted from the light source 21 to pass through one of the separation holes 221 on one side in the Y direction and allows the other part of the illumination light Ls to pass through the other separation hole 221 on the other side in the Y direction. Thus, the illumination light Ls is separated into spectral components. The spectral components of the illumination light Ls guided from the separation holes 221 of the spectroscopic member 22 as an object point will be described as first light components Ls11 and Ls12. The spectral components guided from the slit hole 231 of the first focus optical system 23 as the object point will be described as second light components Ls21 and Ls22.

The spectroscopic member 22, optical scanner 51, and optical path splitter 52 of the illumination system 2 and the anterior segment Ea are positioned to achieve optical conjugation or quasi-optical conjugation. The first light components Ls11 and Ls12 guided by the first illumination system lens 24 substantially form an image on a reflection surface of the optical scanner 51 and are reflected by the optical scanner 51 toward the second illumination system lens 25. Thereafter, the first light components Ls11 and Ls12 are guided to the optical path splitter 52 by the second illumination system lens 25, substantially form an image on an annular reflection surface of the optical path splitter 52, and are reflected by the reflection surface toward the objective lens 53. Each of the first light components Ls 11 and Ls12 condensed by the objective lens 53 form an image on the anterior segment Ea, and then illuminate the fundus Ef.

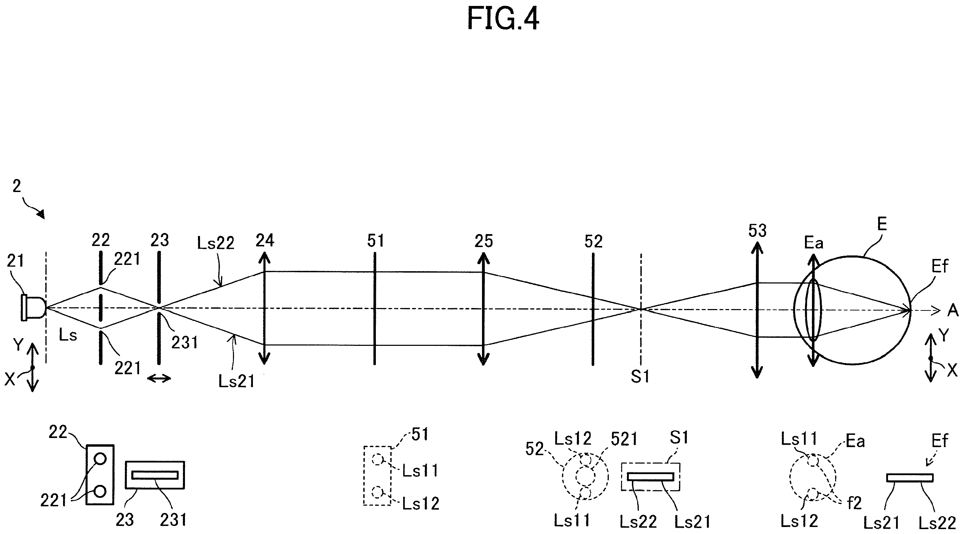

Below the side view 2-2, FIG. 3 (and FIG. 4) shows the optical members, i.e., the spectroscopic member 22, the first focus optical system 23, the optical scanner 51, and the optical path splitter 52, in plan view (the spectroscopic member 22 and the first focus optical system 23 are viewed in the direction of the optical axis A), together with the illumination light Ls (the second light components Ls21 and Ls22) in section taken at a position S1 on the optical path.

FIG. 4 is an optical path diagram of the illumination system 2 schematically showing an optical path of the illumination light Ls (the second light components Ls21, Ls22) passing through the first focus optical system 23. The first focus optical system 23 of the illumination system 2 and the fundus Ef (the target site) are positioned to achieve optical conjugation or quasi-optical conjugation. The second light components Ls21 and Ls22 emitted from the spectroscopic member 22 are guided by the first illumination system lens 24 and reflected by the optical scanner 51 toward the second illumination system lens 25. Thereafter, the second light components Ls21 and Ls22 are guided to the optical path splitter 52 by the second illumination system lens 25 and reflected toward the objective lens 53 by the reflection surface of the optical path splitter 52. Each of the second light components Ls21 and Ls22 forms an image again at the position S1 on the optical path between the optical path splitter 52 and the objective lens 53. The second light components Ls21 and Ls22 collected by the objective lens 53 are condensed on the anterior segment Ea and then illuminates the fundus Ef. The second light components Ls21 and Ls22 substantially form an image again on the fundus Ef. As described above, in the illumination system 2, the optical scanner 51 guides the illumination light Ls including the second light component Ls21 (first spectral component) and the second light component Ls22 (second spectral component) to project the illumination light Ls as the slit light on part of the fundus Ef which is the observation target region 61.

Although FIG. 4 illustrates the optical path of the illumination system 2 in a focused state, the ophthalmologic apparatus 1 is capable of adjusting the focus state as described later considering that the position of the subject's eye E may change every time the ophthalmologic apparatus 1 is used.

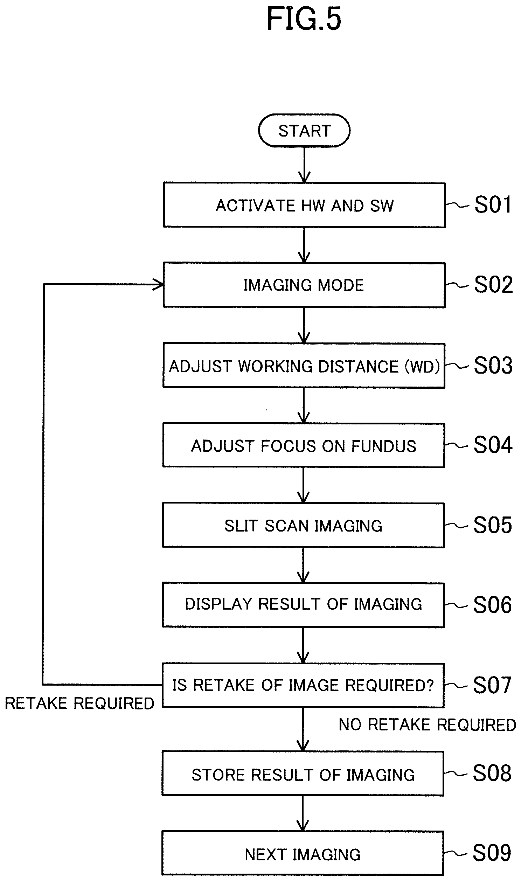

FIG. 5 is an operation flowchart of the ophthalmologic apparatus 1. In Step S01, a user activates the hardware (HW) and software (SW) of the ophthalmologic apparatus 1 via a power switch (not shown).

In Step S02, when receiving an instruction to switch to an imaging mode from the operation unit 12, the control device 14 switches to the imaging mode. In Step S03, the control device 14 adjusts a working distance (WD) to the subject's eye E according to the instruction from the operation unit 12.

In Step 504, the control device 14 performs focus adjustment (also referred to as "focus alignment") of the illumination system 2 and the light receiving system 3. Here, the focus on the fundus Ef which is the target site is adjusted. The ophthalmologic apparatus 1 of the present embodiment performs the focus adjustment including evaluation and control of the focus of the illumination system 2 and the focus of the light receiving system 3 on the fundus Ef before the slit scan imaging of the fundus Ef is performed in Step S05. For the focus evaluation, attention is paid to the fact that the width of the illuminated region R1 (optical image) of the fundus Ef illuminated with the illumination light Ls varies between a state where the illumination system 2 and the light receiving system 3 are focused on the fundus Ef (focused state) and a state where the illumination system 2 and the light receiving system 3 are not focused on the fundus Ef (unfocused state).

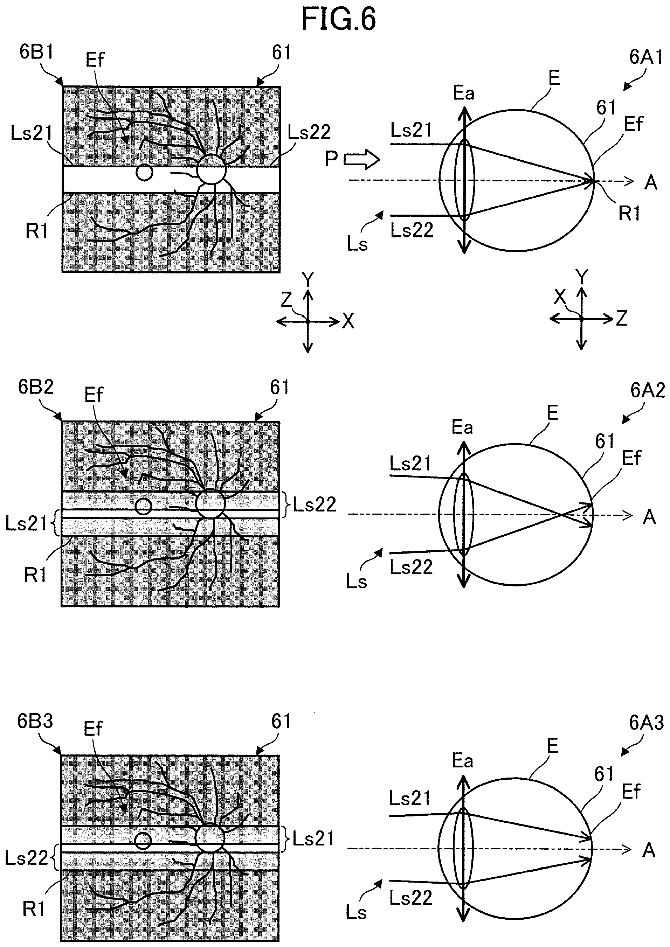

FIG. 6 shows side views 6A1 to 6A3 each illustrating the subject's eye E and the optical path of the illumination light Ls in the focused or unfocused state and front views 6B1 to 6B3 each illustrating the observation target region 61 of the fundus Ef of the subject's eye E illuminated with the illumination light Ls in the focused or unfocused state as viewed from the front (in the P direction).

In the focused state as shown in the side view 6A1, beams of the spectral components (the second light components Ls21 and Ls22) concentrate on the same (or substantially the same) position on the fundus Ef. Thus, as shown in the front view 6B1, the second light components Ls21 and Ls22 are projected on substantially the same position in the Y direction (the scanning direction of the optical scanner 51), projecting the illumination light Ls forming the slit-shaped illuminated region R1. In the unfocused state as shown in the side views 6A2 and 6A3 and the front views 6B2 and 6B3, the beams of the spectral components (the second light components Ls21 and Ls22) concentrate on different positions shifted forward and backward (in the direction of the optical axis A) from the fundus Ef. Thus, the second light component Ls21 (the first spectral component) and the second light component Ls22 (the second spectral component) are projected to form the illuminated region R1 widened (shifted) in the Y direction on the fundus Ef.

For example, when the illumination light Ls forms an image in front of the fundus Ef as shown in the side view 6A2, the second light component Ls21 is projected on a lower position and the second light component Ls22 is projected on an upper position as shown in the front view 6B2, as compared with the second light components Ls21 and Ls22 in the focused state (shown in the front view 6B1). On the other hand, when the illumination light Ls forms an image behind the fundus Ef as shown in the side view 6A3, the second light component Ls21 is projected on an upper position and the second light component Ls22 is projected on a lower position as shown in the front view 6B3, as compared with the second light components Ls21 and Ls22 in the focused state (shown in the front view 6B1). The amount of shift (amount of shift in the direction of the optical axis A) of the imaging position of each of the second light components Ls21 and Ls22 on the fundus Ef may be evaluated from the light intensity I of the illumination light Ls correlated with the amount of shift in the Y direction of the second light components Ls21 and Ls22 in the front views 6B1 to 6B3. In the example of FIG. 6, the illumination light Ls forming the illuminated region R1 in the focused state shown in the front view 6B1 has the higher light intensity I than the illumination light Ls forming the illuminated region R1 in the unfocused state shown in the front view 6B2 or 6B3.

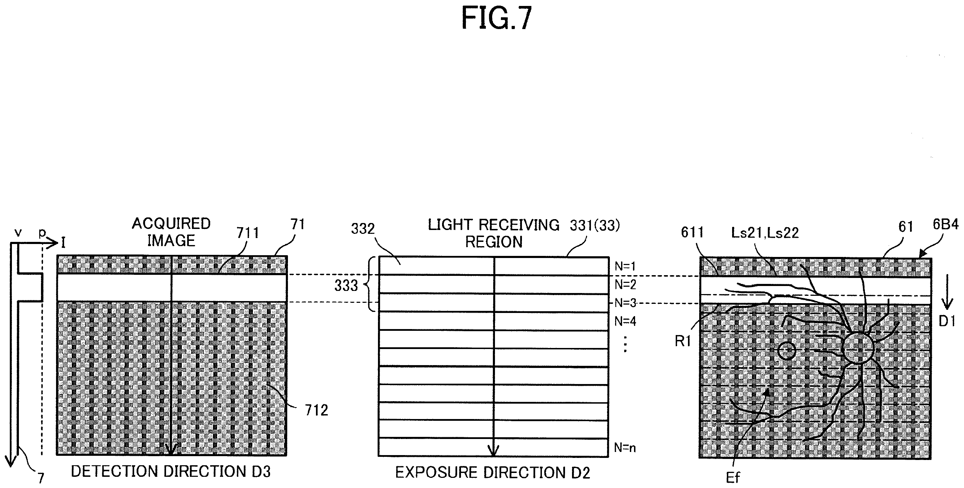

FIG. 7 is a diagram showing a front view 6B4 of the fundus Ef which is the target site, the light receiving region 331 of the imaging element 33, and an acquired image 71 of the fundus Ef taken by the imaging element 33 when the focus adjustment is performed.

The front view 6B4 of the fundus Ef shows that the illumination light Ls is focused on a position above the optical axis A (above in the Y-axis direction). The front view 6B4 of the fundus Ef shows the observation target region 61. The observation target region 61 includes a plurality of imaging target lines 611 formed by dividing the observation target region 61 in the scanning direction D1 of the illumination light Ls. Line numbers N from 1 to n ("n" is an integer of two or more) are given to the imaging target lines 611. The imaging target lines 611 are imaginary regions illustrated for explanatory convenience.

The light receiving region 331 of the imaging element 33 constituted of a CMOS image sensor has light receiving sections that are photodiodes arranged in the up-down and left-right directions to form a matrix. The imaging element 33 is a line exposure imaging element that has the rolling shutter function and includes a plurality of exposure lines 332 in the light receiving region 331. Each of the exposure lines has an imaging position corresponding to an associated one of the imaging target lines 611. The light receiving region 331 has the exposure lines 332 formed by dividing the light receiving region 331 in an exposure direction D2. The exposure lines 332 are unit regions that detect light received in the light receiving region 331 at the same timing. Each of the exposure lines 332 has a plurality of light receiving sections arranged in a line direction orthogonal to the exposure direction D2 in the light receiving region 331 of FIG. 7. Each of the exposure lines 332 has one or more light receiving sections arranged in the exposure direction D2 in the light receiving region 331. Line numbers N from 1 to n ("n" is an integer of two or more) are given to the exposure lines 332. The imaging target line 611 and the exposure line 332 having the same line number N form an image at the corresponding imaging position.

The acquired image 71 is an image formed by the return light Lb that enters and is detected by the light receiving region 331 of the imaging element 33. The acquired image 71 includes a dark region 712 where light detected in the light receiving region 331 has a relatively low light intensity I and a bright region 711 with a high light intensity I where the illumination light Ls corresponding to the illuminated region R1 is detected. The control device 14 obtains, as a detection result, a line profile 7 associated with the light intensity I in a detection direction D3 of the acquired image 71 acquired by the imaging element 33. The line profile 7 indicates the level of the light intensity I in the detection direction D3 which is the same direction as the scanning direction D1 and the exposure direction D2, and includes a first detection value p corresponding to the bright region 711 and a second detection value v corresponding to the dark region 712. In the example of FIG. 7, the illumination light Ls is nearly focused on the fundus Ef, and the intensity ratio of the first detection value p to the second detection value v is relatively high. In the present embodiment, the control device 14 obtains visibility V = (p - v) / (p + v) using the first detection value p at the peak of the light intensity I and the second detection value v at the bottom of the light intensity I in the line profile 7 to evaluate the focused state (the degree of in-focus or out-of-focus). For example, the control device 14 determines that the focus is achieved when the visibility V = (p - v) / (p + v) is equal to or greater than a predetermined threshold value.

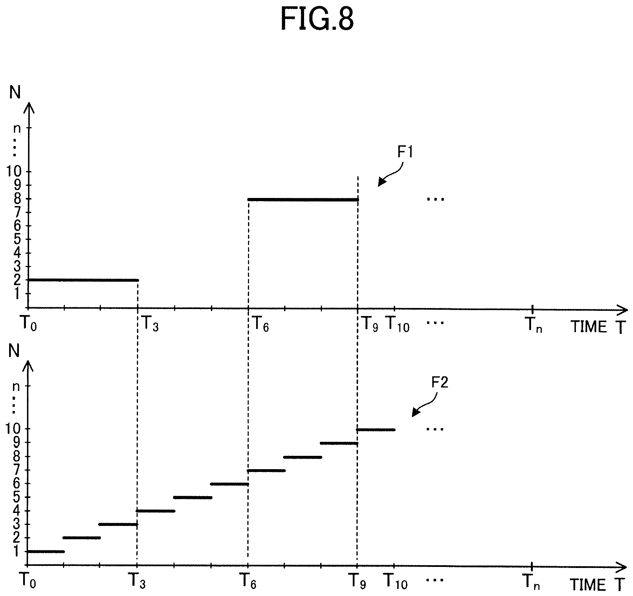

FIG. 8 is a timing chart of the illumination light Ls emitted to the fundus Ef and a timing chart of the exposure operation of the imaging element 33 during the focus adjustment. Timing characteristics F1 related to the emission of the illumination light Ls show that the position to be illuminated changes among the imaging target lines 611 with time T in the scanning direction D1 (see FIG. 7). In the example of FIG. 8, the illumination light Ls (the first and second spectral components) is projected on the position of the imaging target line 611 of the line number N = 2 for a period between a point of time T0 and a point of time T3 and on the position of the imaging target line 611 of the line number N = 8 for a period between a point of time T6 and a point of time T9. The illumination light Ls is not projected (turned off) for a period between a point of time T3 and a point of time T6. Although FIG. 8 illustrates the example in which the illumination light Ls is intermittently emitted in two time periods (the period between the points of time T0 and T3 and the period between the points of time T6 and T9), the illumination light Ls is intermittently emitted also in a period from the point of time T9 to a point of time Tn. Thus, exposure line groups 333 for detecting the second light component Ls21 (first spectral component) and the second light component Ls22 (second spectral component) are also intermittently provided (see also FIG. 11).

Timing characteristics F2 related to the exposure operation of the imaging element 33 show that the position on which the exposure is performed sequentially changes among the exposure lines 332 with time T in the exposure direction D2 (see FIG. 7). In the example of FIG. 8, the exposure operation is sequentially performed on the exposure lines 332 of the line numbers N = 1 to 10 in order in periods divided by the points of time T0 to T10. Also in each of the periods divided by the points of time T10 to Tn, the exposure operation is sequentially performed on the exposure lines 332 of the line numbers N = 11 to n in order.

When the illumination light Ls is emitted and the exposure operation is performed as described above, the illumination light Ls is projected on a substantially central position of the imaging target lines 611 each having the imaging position corresponding to an associated one of the exposure lines 332 (e.g., when the imaging target lines 611 have the line numbers N of "1" to "3," the light is projected on the imaging target line 611 in the middle having the line number N = 2) for a period in which the exposure operation is performed on the exposure lines 332 (e.g., the period between the points of time T0 to T3). In the present embodiment, a group including two or more exposure lines 332 used for the evaluation of the focus of the illumination light Ls is referred to as an exposure line group 333.

As illustrated in FIG. 8, when the exposure operation is sequentially performed on the exposure lines 332 forming a predetermined exposure line group 333 in order in the exposure direction D2, the control device 14 illuminates the illuminated region R1 of the imaging target lines 611 corresponding to the exposure lines 332 forming the exposure line group 333 with the illumination light Ls (the first spectral component (Ls21) and the second spectral component (Ls22)). In the present embodiment, the control device 14 (controller) performs focus determination by obtaining the above-described line profile 7 from the result of the detection by the exposure lines 332 in the exposure line group 333.

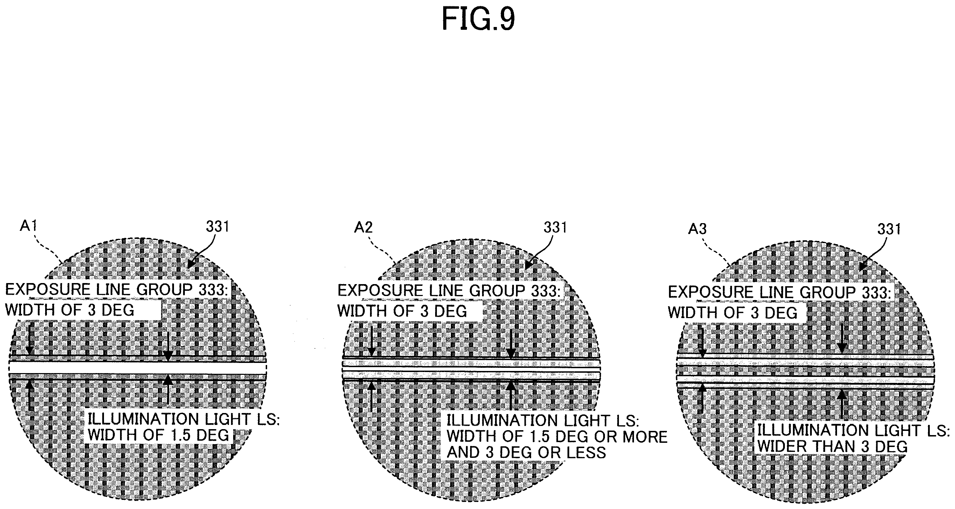

FIG. 9 is a diagram showing a specific example of a relationship between the exposure lines 332 and the illumination light Ls in the focused state or the unfocused state. First, as shown in the light receiving region 331 in the focused state, the exposure line group 333 of the present embodiment has a width of an angle of three [deg] (degrees) in the exposure direction D2 along the optical path from the first focus optical system 23, which is the origin (object point) of the illumination light Ls, to the imaging element 33. In the focused state, the second light components Ls21 and Ls22 that reach the light receiving region 331 have a width of an angle of 1.5 [deg] (degrees) in the exposure direction D2 along the optical axes A and B from the first focus optical system 23 to the imaging element 33.

An example A1 of light reception shows the illumination light Ls in the focused state. The illumination light Ls in the example A1 is projected on a region having a width of about 1.5 [deg] which falls within the exposure line group 333. An example A2 of light reception shows the illumination light Ls in the unfocused state. In the example A2, the illumination light Ls is separated into the second light components Ls21 and Ls22 to some extent. This widens the illumination light Ls in the exposure direction D2 (the up-down direction in FIG. 9) within the exposure line group 333 compared with the illumination light in the example A1, projecting the illumination light Ls on a region slightly narrower than the exposure line group 333. An example A3 of light reception shows the illumination light Ls in the unfocused state. The illumination light Ls in the example A3 is separated into the second light components Ls21 and Ls22 that are projected on separated regions around the boundaries in the widthwise direction of the exposure line group 333. The light intensity I of the illumination light Ls detected by the light receiving region 331 (the first detection value p) decreases in the order of the example A1, the example A2, and the example A3. As thus described, setting of the width of the exposure line group 333 greater in advance than the width of the illumination light Ls in the focused state allows for detection of the illumination light Ls having a width within the width of the exposure line group 333 even with the illumination light Ls being unfocused and widened when separated into the second light components Ls21 and Ls22.

Note that the width of "3 [deg]" of the exposure line group 333 and the width of "1.5 [deg]" of the illumination light Ls are merely examples. As shown in the example A1, the exposure line group 333 may have any width greater than the width of the illumination light Ls in the focused state.

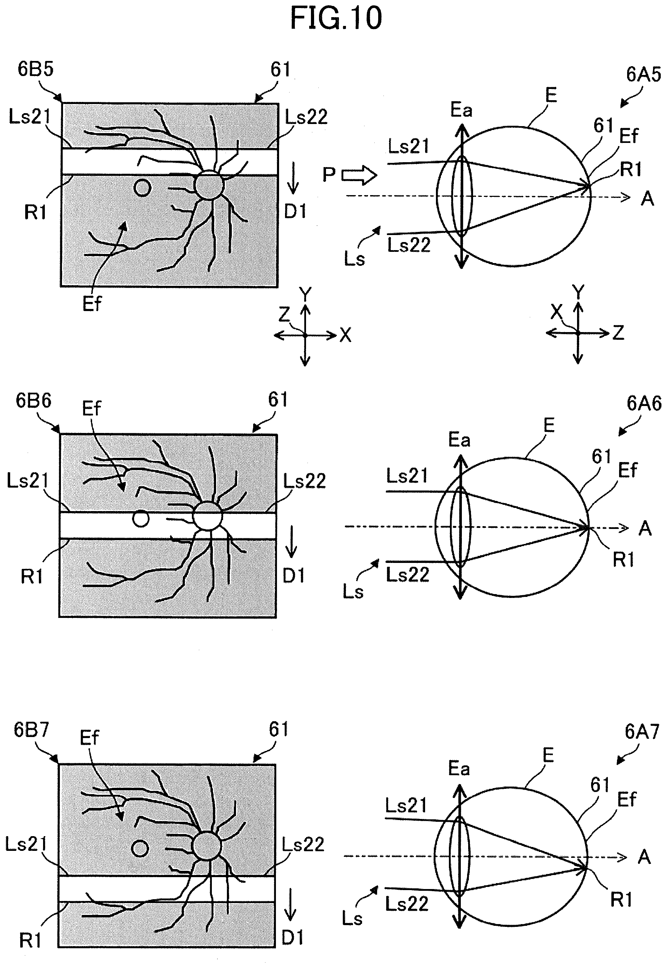

FIG. 10 shows side views 6A5 to 6A7 each illustrating the subject's eye E and the optical path of the illumination light Ls during image capturing for the focus evaluation and front views 6B5 to 6B7 each illustrating the fundus Ef of the subject's eye E illuminated with the illumination light Ls as viewed from the front (in the P direction) during image capturing for the focus evaluation. The control device 14 acquires the image 71 by capturing an image of the return light Lb from the fundus Ef illuminated with the illumination light Ls with the imaging element 33 while changing the deflection angle of the illumination light Ls deflected by the optical scanner 51. Based on the line profile 7 obtained from the acquired image 71, the control device 14 controls the focus of the first and second focus optical systems 23 and 31 so that the visibility V is maximized.

The evaluation and control of the focus are mainly performed by the illumination control unit 141, deflection control unit 142, imaging control unit 143, signal acquisition unit 144, image generation unit 145, focus evaluation unit 146, repetition control unit 147, and focus control unit 148 of the control device 14 illustrated in FIG. 2.

The illumination control unit 141 causes the illumination system 2 to emit the illumination light Ls (e.g., near-infrared light) during the focus evaluation. Use of the near-infrared light as the illumination light Ls allows for reduction of miosis of the subject's eye E.

The signal acquisition unit 144 sequentially acquires the imaging signal outputted from the light receiving region 331 of the imaging element 33 during the rolling shutter driving of the imaging element 33 for the focus evaluation.

The image generation unit 145 generates the acquired image 71 based on the imaging signal acquired by the signal acquisition unit 144 during the rolling shutter driving of the imaging element 33 for the focus evaluation. As shown in FIG. 6, the illumination light Ls is detected as a narrow pattern image in the observation target region 61 in the focused state (see the front view 6B1), and the illumination light Ls is detected as a pattern image widened or separated in the observation target region 61 in the unfocused state (see the front views 6B2 and 6B3).

The focus evaluation unit 146 controls the optical scanner 51 via the illumination control unit 141 and controls the imaging element 33 via the imaging control unit 143 to capture an image. For example, the focus evaluation unit 146 controls, via the deflection control unit 142, the deflection angle of the illumination light Ls deflected by the optical scanner 51 so that the illumination light Ls illuminates the fundus Ef. The focus evaluation unit 146 obtains the line profile 7 from the acquired image 71 and evaluates the focus based on the line profile 7.

While changing the positions of the focus lenses of the first and second focus optical systems 23 and 31, the repetition control unit 147 performs repetition control to cause the focus evaluation unit 146, the signal acquisition unit 144, and the image generation unit 145 to operate repeatedly for each of different positions of the first focus optical system 23. Thus, the acquired image 71 for each position of the first focus optical system 23 is obtained. When the first and second focus optical systems 23 and 31 include varifocal lenses instead of the focus lenses, the repetition control unit 147 performs the repetition control for each of different focal positions of the varifocal lenses.

The focus control unit 148 controls the focus of the first focus optical system 23 and the focus of the second focus optical system 31 to cause the illumination system 2 and the light receiving system 3 to focus on the fundus Ef. As described above, the focusing of the light receiving system 3 by the second focus optical system 31 and the focusing of the illumination system 2 by the first focus optical system 23 occur in synchronization in accordance with the diopter (visibility) of the subject's eye E. The focus control unit 148 controls the first and second focus optical systems 23 and 31 based on the line profile 7 obtained from the acquired image 71 to maximize the visibility V (i.e., to bring the systems closest to the focused state).

Thus, when the first focus optical system 23 is focused on the target site of the subject's eye E (the fundus Ef in the present embodiment), the second focus optical system 31 performs control so that the imaging element 33 is focused on the target site in synchronization with the focusing of the first focus optical system 23. The control device 14 is capable of causing the imaging element 33 to detect the illuminated region R1 of the fundus Ef illuminated with the illumination light Ls (the second light components Ls21 and Ls22) thereby evaluating the line profile 7 so as to perform the evaluation and control of the focus.

As shown in FIG. 11, the focus evaluation unit 146 is capable of evaluating the focus by obtaining the line profile 7 from the acquired image 71 including the bright regions 711 obtained from the timing chart of FIG. 8 and calculating the visibility V from the line profile 7. The focus evaluation unit 146 may perform the focus evaluation based on the line profile 7 including the first detection value p corresponding to one bright region 711 or the line profile 7 including the first detection values p corresponding to two or more bright regions 711. When the line profile 7 including two or more bright regions 711 is used, the control device 14 is capable of determining that the position of the bright region 711 having the maximum first detection value p among the bright regions 711 is the most focused position. Thus, for example, when the bright region 711 having the high first detection value p is observed at the substantially central position in the fundus Ef, the control device 14 is capable of determining that the focal point is located deep behind the entire fundus Ef which is the observation target region 61. When the bright region 711 having the high first detection value p is observed at the starting end or terminal end of the fundus Ef in the scanning direction D1, the control device 14 is capable of determining that the focal point is located on the front side of the entire fundus Ef which is the observation target region 61. This allows the control device 14 to determine whether the focal point needs to be moved forward or rearward of the fundus Ef to enable the focusing on the fundus Ef.

Referring back to FIG. 5, the control device 14 performs the slit scan imaging in Step S05. In the slit scan imaging, the illumination control unit 141, deflection control unit 142, imaging control unit 143, signal acquisition unit 144, image generation unit 145, and display control unit 149 of the control device 14 shown in FIG. 2 are mainly operated.

The illumination control unit 141 controls the emission of the illumination light Ls from the light source 21 (i.e., the illumination system 2). The illumination control unit 141 causes the light source 21 to emit visible light as the illumination light Ls during the slit scan imaging.

The deflection control unit 142 controls the deflection angle of the illumination light Ls deflected by the optical scanner 51. For the slit scan imaging, the deflection control unit 142 controls the optical scanner 51 to deflect the illumination light Ls in the Y direction so that the illumination light Ls (slit light) scans the inside of the fundus Ef in the Y direction which is the widthwise direction of the slit light (e.g., from top to bottom in the scanning direction D1).

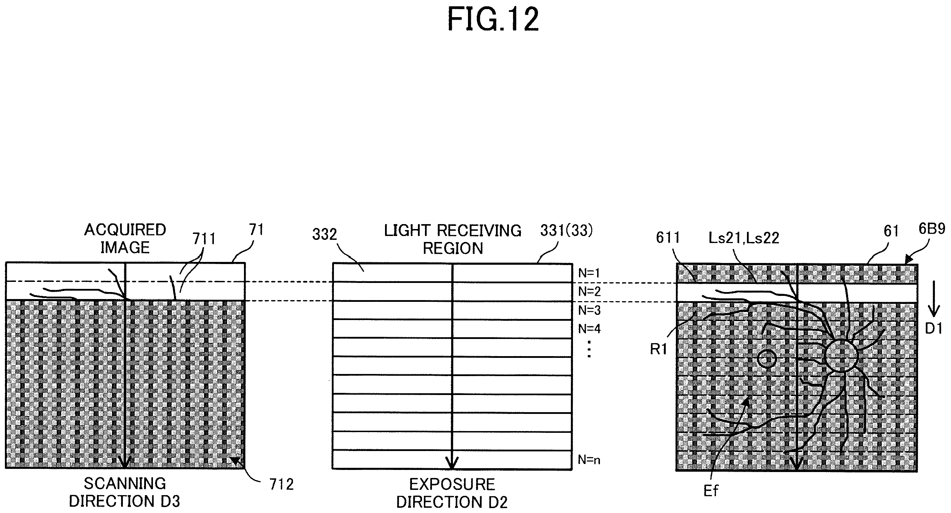

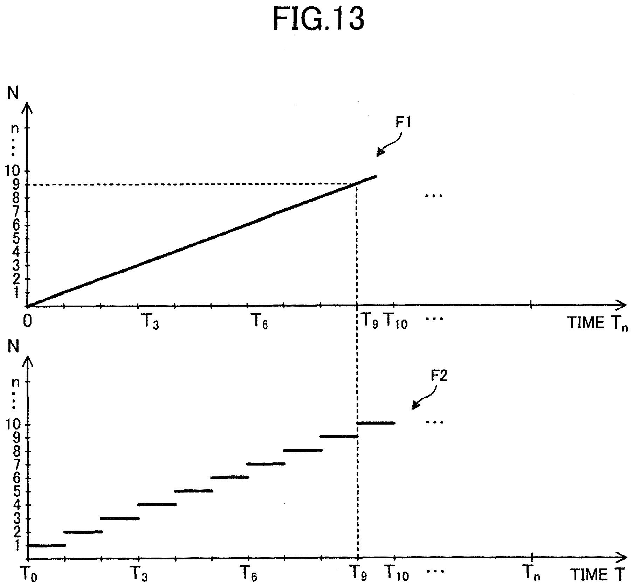

As shown in FIG. 12, the illuminated region R1 illuminated with the illumination light Ls moves in the Y direction in the fundus Ef (the target site) according to the deflection of the illumination light Ls in the Y direction (see also the side views 6A5 to 6A7 and the front views 6B5 to 6B7 of FIG. 10). Although it is illustrated in FIG. 12 that the illuminated region R1 illuminated with the illumination light Ls has substantially the same width as the imaging target line 611, the illuminated region R1 may be wider than the imaging target line 611. As shown in FIG. 13, the position of the exposure line 332 for the exposure of the return light Lb in the light receiving region 331 also moves in the exposure direction D2 (Y direction) in synchronization with the movement of the illuminated region R1.

The imaging control unit 143 controls the driving of the imaging element 33. During the slit scan imaging, the imaging control unit 143 drives the imaging element 33 to perform the rolling shutter function when the optical scanner 51 is deflecting the illumination light Ls in the Y direction (i.e., during the movement of the illuminated region R1 in the scanning direction D1 in the fundus Ef).

Specifically, the imaging control unit 143 allows continuous detection of the return light Lb by the exposure line 332 while causing the exposure line 332 to follow the illuminated region illuminated with the return light Lb moving in the scanning direction D1 in the light receiving region 331. In other words, the illuminated region R1 is continuously detected while the imaging element 33 causes the exposure range to locally follow the movement of the illuminated region R1 in the scanning direction D1 in the fundus Ef. The rolling shutter driving is achieved by a known technique, and thus will not be described in detail below.

The signal acquisition unit 144 is wired or wirelessly connected to the imaging element 33 via a communication interface (not shown). The signal acquisition unit 144 sequentially acquires an imaging signal (also referred to as a detection signal or a light reception signal) from the light receiving region of the imaging element 33 when the optical scanner 51 is deflecting the illumination light Ls during the slit scan imaging.

The image generation unit 145 is capable of generating a fundus image based on the imaging signal acquired by the signal acquisition unit 144 when the optical scanner 51 is deflecting the illumination light Ls during the slit scan imaging. The acquired image 71 of FIG. 12 shows a state in which the light components detected in the exposure lines 332 of line numbers N = 1 and N = 2 are superimposed to show part of the image of the fundus Ef.

In Step S06, the display control unit 149 controls the contents shown on the display 13. For example, in the slit scan imaging, the display control unit 149 causes the display 13 to show the image of the fundus Ef generated by the image generation unit 145.

In Step S07, the control device 14 determines whether the retake of the image is required. The control device 14 executes the process of Step S02 when retaking the image ("retake required" in Step S07), and executes the process of Step S08 is when retaking no image ("no retake required" in Step S07). In Step S07, the control device 14 may determine whether or not to retake the image based on a selection instruction inputted to the operation unit 12 by the user. The user is able to determine whether or not to retake the image by checking the acquired image shown on the display 13.

In Step S08, the control device 14 stores the image acquired by the slit scan imaging as the imaging result in a storage unit (or a storage device) which is not shown.

In Step S09, the control device 14 proceeds to the next imaging (e.g., taking an image of another subject's eye E) in accordance with an instruction inputted to the operation unit 12.

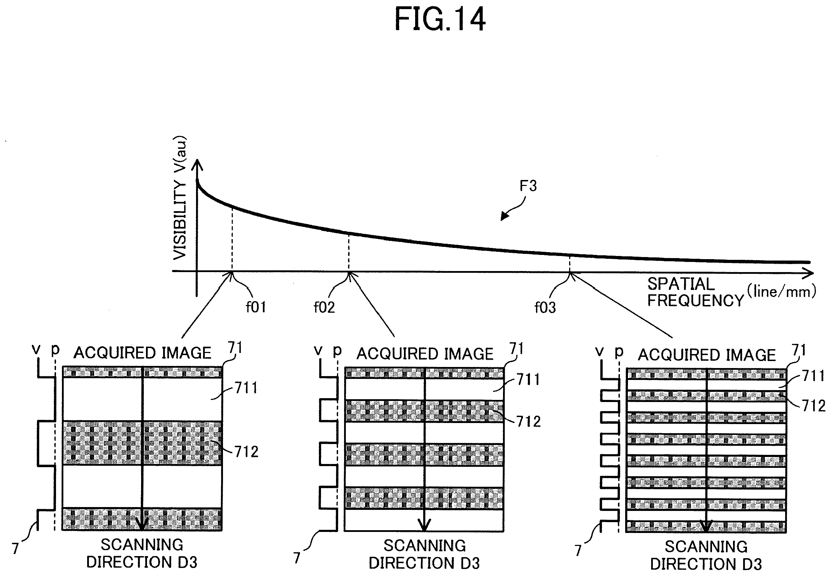

The ophthalmologic apparatus 1 of the present embodiment described above may be configured to obtain a modulation transfer function (MTF). FIG. 14 is a diagram illustrating a relationship between the line profile 7 and a modulation transfer function F3.

The modulation transfer function F3 represents the visibility V [au] for each spatial frequency [line / mm] of the bright region 711 and the dark region 712 that are periodically detected in the acquired image 71. The control device 14 is capable of obtaining the modulation transfer function F3 by obtaining the visibility V in the focused state in advance at different spatial frequencies f01 to f03 (by increasing or decreasing the number of exposure lines 332 forming each exposure line group 333 and the interval between the exposure line groups). The modulation transfer function F3 represents the relationship between the visibility V and the spatial frequencies in an ideal state of an optical system having a certain capability. The visibility V at a certain spatial frequency is measured under the influence of the optical system of the subject's eye E (including the cornea and the crystalline lens), and is thus not comparable to an ideal visibility V although the optical system is focused (is in focus) on the subject's eye E (fundus Ef). The light projected on the subject's eye E is captured by the imaging element 33 via the optical system having the modulation transfer function F3.

It is possible to interpret a difference between the visibility V measured in a state considered as the ideal state and the visibility V actually measured as a change caused by the optical system of the subject's eye E (e.g., the cornea and the crystalline lens). Thus, obtaining the modulation transfer function F3 in advance allows the control device 14 to measure parameters of the modulation transfer function F3 related to the subject's eye E.

The control device 14 is also capable of using the degree of contrast of the modulation transfer function F3 (e.g., the difference between the visibility V measured in a state considered as the ideal state and the visibility V actually measured) as a clue for determining whether the optical system is focused (is in focus) on the subject's eye E. Hence, use of the modulation transfer function F3 facilitates focus determination.

Further, the control device 14 is capable of estimating and obtaining an appropriate threshold value of the visibility V used for the focus determination for each spatial frequency from the line profile 7 obtained at a specific spatial frequency. The threshold value of the visibility V for the focus determination is set manually or automatically. This configuration allows the control device 14 to perform the focus determination based on a plurality of line profiles 7 by increasing or decreasing the number of exposure lines 332 forming each exposure line group 333 and the interval between the exposure line groups.

As has been described above, according to the present embodiment, the ophthalmologic apparatus 1 includes: the spectroscopic member that separates light emitted from the light source 21 into the first spectral component (Ls21) and the second spectral component (Ls22); the optical scanner 51 that guides the first spectral component (Ls21) and the second spectral component (Ls22) to the observation target region 61 of the subject's eye E including a plurality of imaging target lines 611 formed by dividing the observation target region 61 in the scanning direction D1; the line exposure imaging element 33 that includes a plurality of exposure lines 332 capable of detecting the return light Lb from the observation target region 61 in the light receiving region 331, each of the exposure lines 332 having an imaging position corresponding to an associated one of the imaging target lines 611; and the control device 14 (controller) that illuminates, when the exposure operation is sequentially performed on two or more of the exposure lines 332 forming a predetermined exposure line group 333 in order, a region of the imaging target lines 611 corresponding to the two or more exposure lines 332 forming the exposure line group 333 with the first spectral component (Ls21) and the second spectral component (Ls22) to perform focus determination based on the result of detection by the exposure lines 332 in the exposure line group 333.

This configuration allows for reduction of the number of optical members for separating the illumination light Ls, for example, so that the illumination system 2 no longer requires other optical members than the spectroscopic member 22 for separating the illumination light Ls such as a prism, thereby making it possible to use the illumination system 2 including the light source 21 both as a light source and as an optical path for the focus evaluation and a light source and an optical path for the observation. Therefore, this allows for providing the ophthalmologic apparatus 1 and the focus determination method that enable the observation of the subject's eye E and the focus evaluation with a simple configuration.

Although the embodiments of the present disclosure have been described above, the aspects of the present disclosure are not limited to the configurations in the embodiments.

For example, as has been described in the present embodiment, the spectroscopic member 22 two separation holes 221 are arranged apart from each other in the Y direction of FIG. 1. However, the number and arrangement of the separation holes 221 may be changed as long as a plurality of separation holes 221 are arranged eccentric to the optical axis A.

The spectroscopic member 22, the first focus optical system 23, and the imaging element 33 may be rotatable in synchronization about the optical axes A and B. In this case, for example, the reflection surface of the optical scanner 51 may be configured to be freely tiltable in two axial directions, for example, two axial directions perpendicular to the optical axis. Thus, the scanning direction of the illumination light Ls on the fundus Ef as the target site may be any direction, and the orientation of the slit-shaped illumination light Ls, for example, the orientation of the longitudinal direction of the illumination light Ls, may be changed. This allows more accurate observation of the target site when the illumination light Ls is projected on the illumined region R1 from a different angle. In addition to the spectroscopic member 22, the first focus optical system 23, and the imaging element 33, the optical scanner 51 may be synchronously rotated (e.g., about a normal line of a reflector (reflection surface) of the optical scanner 51) to change the scanning direction of the illumination light Ls on the fundus Ef and the orientation of the illumination light Ls.

The first spectral component (Ls21) and the second spectral component (Ls22) may be emitted as spectral components having different characteristics. For example, making the first spectral component (Ls21) and the second spectral component (Ls22) have different frequencies allows for evaluation as to whether the apparatus body 11 is located close to or far from the observation target region based on the vertical positions of the first spectral component (Ls21) and the second spectral component (Ls22) projected on the target to be observed. The control device 14 is capable of determining that the apparatus body 11 is far from the observation target region when the first spectral component (Ls21) of the illumination light Ls projected on the fundus Ef as the observation target region is located below the second spectral component (Ls22) as can be seen in the front view 6B2 in FIG. 6. The control device 14 is capable of determining that the apparatus body 11 is far from the observation target region when the first spectral component (Ls21) is located above the second spectral component (Ls22) as can be seen in the front view 6B3 in FIG. 6. Examples of a spectroscopic method using the first spectral component (Ls21) and the second spectral component (Ls22) having different wavelengths may include arrangement of a dichroic filter in the separation holes 221 of the spectroscopic member 22 only during the focus evaluation.

## Claims

1. An ophthalmologic apparatus (1), **characterized by** comprising:
a spectroscopic member (22) that separates light emitted from a light source (21) into a first spectral component (Ls21) and a second spectral component (Ls22);
an optical scanner (51) that guides the first spectral component and the second spectral component to an observation target region (61) of a subject's eye (E) including a plurality of imaging target lines (611) formed by dividing the observation target region in a scanning direction (D1);
a line exposure imaging element (33) that includes a plurality of exposure lines (332) capable of detecting return light (Lb) from the observation target region in a light receiving region (331, 331A), each of the exposure lines (332) having an imaging position corresponding to an associated one of the imaging target lines; and
a controller (14) that illuminates, when exposure operation is sequentially performed on two or more of the exposure lines forming a predetermined exposure line group (333) in order, a region of the imaging target lines corresponding to the two or more exposure lines forming the predetermined exposure line group with the first spectral component and the second spectral component to perform focus determination based on a result of detection by the exposure lines in the exposure line group.

2. The ophthalmologic apparatus (1) of claim 1, wherein
the controller (14)
obtains, as the result of detection, a line profile (7) including light intensity associated with a detection direction (D3) in which an image is acquired by the imaging element, and
determines that focus is achieved when the line profile indicates that visibility V = (p - v) / (p + v), where p represents a first detection value at the peak of the light intensity and v represents a second detection value at the bottom of the light intensity, is equal to or greater than a predetermined threshold value.

3. The ophthalmologic apparatus (1) of claim 2, wherein
the exposure line group (333) for detecting the first spectral component and the second spectral component includes a plurality of exposure line groups (333) that are provided intermittently.

4. The ophthalmologic apparatus (1) of claim 3, wherein
the controller (14) performs focus evaluation based on the line profile including a plurality of line profiles obtained by increasing or decreasing the number of the exposure lines forming each exposure line group and an interval between the plurality of exposure line groups.

5. The ophthalmologic apparatus (1) of any one of claims 1 to 4, wherein
the exposure line group (333) for detecting the first spectral component and the second spectral component includes a plurality of exposure line groups (333) that are provided intermittently.

6. The ophthalmologic apparatus (1) any one of claim 1 to 5, wherein
the controller (14) performs the focus determination based on a plurality of line profiles obtained by increasing or decreasing the number of the exposure lines forming the exposure line group including a plurality of exposure line groups and an interval between the plurality of exposure line groups.

7. The ophthalmologic apparatus (1) of any one of claims 1 to 6, wherein
an illuminated region (R1) illuminated with the first spectral component and the second spectral component corresponds to the plurality of the exposure lines.

8. A focus evaluation method for an ophthalmologic apparatus (1), the ophthalmologic apparatus (1) including:
a spectroscopic member (22) that separates light emitted from a light source (21) into a first spectral component (Ls21) and a second spectral component (Ls22);
an optical scanner (51) that guides the first spectral component and the second spectral component to an observation target region (61) of a subject's eye (E) including a plurality of imaging target lines (611) formed by dividing the observation target region in a scanning direction (D1); and
a line exposure imaging element (33) that includes a plurality of exposure lines (332) capable of detecting return light (Lb) from the observation target region in a light receiving region (331, 331A), each of the exposure lines (332) having an imaging position corresponding to an associated one of the imaging target lines, the focus determination method **characterized by** comprising:
illuminating, when exposure operation is sequentially performed on two or more of the exposure lines forming a predetermined exposure line group (333) in order, a region of the imaging target lines corresponding to the two or more exposure forming the predetermined exposure line group with the first spectral component and the second spectral component to perform focus determination based on a result of detection by the exposure lines in the exposure line group.
